# EUROPÄISCHE PATENTSCHRIFT

(11) **EP 1 244 607 B1**
(45) Veröffentlichungstag und Bekanntmachung des Hinweises auf die Patenterteilung: **17.09.2003**
(21) Anmeldenummer: 00989970.9
(22) Anmeldetag: 11.12.2000
(51) Int. Cl.: C07C 39/16

(54) **FLUIDISIERTER BISPHENOL-STAUB**
FLUIDISED BISPHENOL DUST
POUDRE DE BISPHENOL FLUIDISEE

(30) Priorität: 23.12.1999 DE 19962530
(43) Veröffentlichungstag der Anmeldung: 02.10.2002
(73) Patentinhaber: Bayer Aktiengesellschaft, 51368 Leverkusen (DE)
(72) Erfinder: BÖDIGER, Michael, League City, TX 77573 (US); NEUMANN, Rainer, 47803 Krefeld (DE); KOSTYSZYN, Nikolai, 47906 Kempen (DE); LANZE, Rolf, 47804 Krefeld (DE); HEYDENREICH, Frieder, 40593 Düsseldorf (DE); PREIN, Michael, B-2930 Brasschaat (BE)
(86) Internationale Anmeldenummer: EP0012482
(87) Internationale Veröffentlichungsnummer: WO01047851

(56) Entgegenhaltungen:
- EP-A- 0 278 246
- CH-A- 493 445

## Beschreibung

Die vorliegende Erfindung betrifft fluidisierten Bisphenol-Staub enthaltend Bisphenol-Staub und Wasser, ein Verfahren zu dessen Herstellung sowie ein Verfahren zur Herstellung von einem Bisphenol, wobei dem Verfahren fluidisierter Bisphenol-Staub zugeführt wird.

Bis(4-hydroxyaryl)alkane, im Folgenden Bisphenole genannt, sind als Ausgangsstoffe oder als Zwischenprodukte zur Herstellung einer Vielzahl kommerzieller Produkt von Bedeutung. Bisphenole können durch die Kondensation von Phenolen und Carbonylverbindungen hergestellt werden. Dabei können substituierte Phenole oder unsubstituiertes Phenol verwendet werden.

Von besonderer technischer Bedeutung ist das Kondensationsprodukt aus der Reaktion zwischen Phenol und Aceton, 2,2-Bis(4-hydroxyphenyl)propan (Bisphenol A, BPA). BPA dient als Ausgangstoff zur Herstellung verschiedenartiger polymerer Werkstoffe wie beispielsweise Polyarylate, Polyetherimide, Polysulfone und modifizierter Phenol-Formaldehydharze. Bevorzugte Anwendungsgebiete liegen in der Herstellung von Epoxyharzen und Polycarbonaten.

Verfahren zur Herstellung von Bisphenolen durch sauer katalysierte Umsetzung von Phenolen mit Carbonylverbindungen sind beispielsweise bekannt aus US-A 2 775 620 und aus EP-A-0 342 758.

Bisphenole allgemeiner Struktur können nach Verfahren hergestellt werden, die den Verfahren zur Herstellung von BPA analog sind.

Bisphenole und insbesondere Bisphenol-A werden vorteilhaft in die Form von sogenannten Prills (Prills sind kugelförmige Feststoffteilchen, die durch das Erstarren von Schmelzetropfen in einem Gasstrom hergestellt werden können) oder Schuppen überführt. Dadurch wird eine einfache Lagerung, ein einfacher Transport und eine einfache Handhabung möglich.

Bei der Produktion von Bisphenol-Prills und Bisphenol-Schuppen oder bei anderweitiger Handhabung von Bisphenolen fällt ein feiner Bisphenol-Staub an, der von hoher Reinheit bezüglich des Bisphenols ist. Der Staub ist klebrig und schwer handhabbar. Seine Handhabung beinhaltet die Gefahr von Staubexplosionen. Aus diesem Grunde wird der Bisphenol-Staub üblicherweise unter hohem manuellem Handhabungsaufwand und umfangreichen Sicherheitsvorkehrungen wegen der Staubexplosionsgefahr entsorgt und beispielsweise verbrannt. Dies hat den Nachteil, dass die Entsorgung des Staubes eine Vernichtung des wertvollen Rohstoffes Bisphenol darstellt, die zudem wegen des hohen Handhabungsaufwandes unwirtschaftlich ist.

Der vorliegenden Erfindung liegt daher die Aufgabe zugrunde ein Verfahren zur Vereinfachung der Handhabbarkeit von Bisphenol-Staub sowie eine Möglichkeit zu dessen wirtschaftlicher Verwertung bereitzustellen.

Die erfindungsgemäße Aufgabe wird gelöst durch Bereitstellung eines Verfahrens zur Fluidisierung von Bisphenol-Staub. Dieses Verfahren ermöglicht die Zuführung des Bisphenol-Staubes in das Verfahren zur Herstellung von Bisphenolen.

Gegenstand der vorliegenden Erfindung ist daher ein Verfahren zur Fluidisierung von Bisphenol-Staub, umfassend die Zugabe von 0,1 bis 10 Gewichtsteilen Wasser pro 1 Gewichtsteil Bisphenol Staub.

Gegenstand der vorliegenden Erfindung ist weiterhin fluidisierter Bisphenol-Staub enthaltend 0,1 bis 10 Gewichtsteile Wasser je 1 Gewichtsteil Bisphenol-Staub.

Gegenstand der vorliegenden Erfindung ist weiterhin ein Verfahren zur Herstellung von einem Bisphenol, wobei dem Verfahren vor, während oder nach der Reaktion eines Phenols mit einer Carbonylverbindung fluidisierter Bisphenol-Staub enthaltend 0,1 bis 10 Gewichtsteile Wasser je 1 Gewichtsteil Bisphenol-Staub zugeführt wird.

Erfindungsgemäße Bisphenole sind beliebige Bisphenole. Bevorzugt ist Bisphenol A.

Erfindungsgemäße Phenol sind beliebig substituierte Phenole, bevorzugt ist unsubstituiertes Phenol.

Erfindungsgemäße Carbonylverbindungen sind beliebige Carbonylverbindungen. Bevorzugt ist Aceton.

Die erfindungsgemäß eingesetzte Wassermenge beträgt 0,1 bis 10 Gewichtsteile, bevorzugt 0,15 bis 1 Gewichtsteil, besonders bevorzugt 0,2 bis 0,4 Gewichtsteile pro 1 Gewichtsteil Bisphenol.

Der fluidisierte Bisphenol-Staub kann vorzugsweise ohne Abtrennung von Wasser dem Verfahren zur Herstellung von Bisphenol zugeführt werden.

Der fluidisierte Bisphenol-Staub wird bevorzugt vor der Zuführung in das Verfahren zur Herstellung von Bisphenol, vorzugsweise in einem Phenol, aufgelöst. Dieses Phenol ist bevorzugt dasselbe Phenol, das im Verfahren zur Herstellung des Bisphenols eingesetzt wird.

Die Zuführung des fluidisierten Bisphenol-Staubes in das Verfahren zur Herstellung von Bisphenol kann an beliebiger Stelle des Verfahrens zur Herstellung von Bisphenol erfolgen. Vorzugsweise erfolgt die Zuführung in die Kristallisationsstufe des Verfahrens zur Herstellung von Bisphenol. Eine weitere bevorzugte Ausführungsform des Verfahrens ist diejenige bei der die Zuführung des fluidisierten Bisphenol-Staubes in die Stufe der Mutterlaugenentwässerung des Verfahrens zur Herstellung von Bisphenol erfolgt.

In einer bevorzugten Ausführungsform der vorliegenden Erfindung hat der Bisphenol-Staub eine Teilchengröße von weniger als 1 mm, besonders bevorzugt von weniger als 0,5 mm, ganz besonders bevorzugt weniger als 50 µm.

Erfindungsgemäß haben die Partikel, die den Bisphenol-Staub bilden, beliebige Gestalt. Bevorzugt haben sie die Gestalt von Bruchstücken geometrischer Formen, beispielsweise Bruchstücken von Plättchen oder Kugeln.

Erfindungsgemäß ist unter Fluidisierung das Herbeiführen von Fließfähigkeit zu verstehen. Fließfähigkeit in diesem Zusammenhang bedeutet beispielsweise, dass der fluidisierte Bisphenol-Staub, allein durch die Schwerkraft getrieben, durch ein 50 cm langes Rohr mit einem Durchmesser von 20 cm mit ausreichend schneller Geschwindigkeit, bevorzugt mit einer Geschwindigkeit von größer als 300 kg pro 10 Minuten fließt.

Der erfindungsgemäße Bisphenol-Staub hat bevorzugt eine Reinheit von größer als 95 Gew.-%.

Der erfindungsgemäße Bisphenol-Staub kann auch Isomere und Nebenprodukte, die bei der Bisphenolherstellung entstehen enthalten.

Insbesondere ist sogenannte "Anfahrware", die beim Anfahren der Bisphenol A-Herstellung staubförmig anfällt, ebenfalls unter dem Begriff Bisphenol-Staub zu fassen.

Das erfindungsgemäße Verfahren zur Herstellung von einem Bisphenol, wobei dem Verfahren vor, während oder nach der Reaktion eines Phenols mit einer Carbonylverbindung fluidisierter Bisphenol-Staub zugeführt wird, wird bevorzut so durchgeführt, dass die Menge des dem Verfahren zugeführten Bisphenol-Staubes wesentlich geringer ist, als die Menge des insgesamt durch das Verfahren hergestellten Bisphenols. Bevorzugt ist die Menge des zugeführten Bisphenol-Staubes kleiner als 10 % der Menge des durch das Verfahren hergestellten Bisphenols.

Das erfindungsgemäße Verfahren hat zahlreiche Vorteile. Der mit Wasser fluidisierte Bisphenol-Staub emittiert bei seiner Handhabung keinen Staub in die Umgebungsluft wie dies nicht fluidisierter Bisphenol-Staub bei seiner Handhabung tut. Dadurch ist die Gefahr von Staubexplosionen nicht mehr gegeben. Durch die Möglichkeit der Zuführung des fluidisierten Bisphenol-Staubes in das Verfahren zur Herstellung von Bisphenol entfällt die aufwendige und teure Entsorgung des Staubes. Der wertvolle Rohstoff Bisphenol wird zurückgewonnen.

Das erfindungsgemäße Verfahren zur Herstellung von BPA beruht bevorzugt auf der säurekatalysierten Umsetzung von Phenol mit Aceton, wobei bevorzugt ein Mengenverhältnis Phenol:Aceton von größer als 5 : 1 in der Reaktion eingestellt wird. Als saure Katalysatoren können homogene wie auch heterogene Brönstedsäuren oder Lewissäuren genutzt werden, so beispielsweise starke Mineralsäuren wie Salzsäure oder Schwefelsäure. Bevorzugt kommen gelförmige oder makroporöse sulfonierte vernetzte Polystyrolharze (saure lonentauscher) zum Einsatz. Die nachfolgenden Ausführungen beziehen sich auf ein Verfahren zur Herstellung unter Nutzung von sauren Ionentauschern als Katalysatoren.

Zur Erzielung hoher Selektivitäten kann die Umsetzung von Phenol mit Aceton in Gegenwart geeigneter Mercaptoverbindungen als Cokatalysatoren durchgeführt werden. Diese können entweder homogen in der Reaktionslösung gelöst sein oder über ionische oder kovalente Bindungen an der sulfonierten Poylstyrolmatrix fixiert werden. Die Reaktionseinheit ist bevorzugt ein Schichtbett oder Wirbelbett, die aufoder abwärts durchflossen werden, oder eine Kolonne nach Art einer Reaktivdestillationskolonne.

Bei der Umsetzung von Phenol mit Aceton in Gegenwart saurer Katalysatoren und Mercaptoverbindungen als Cokatalysatoren entsteht eine Produktmischung, die neben nicht umgesetztem Phenol und gegebenenfalls Aceton in erster Linie BPA und Wasser enthält. Daneben treten in geringen Mengen typische Nebenprodukte der Kondensationreaktion auf, so beispielsweise 2-(4-hydroxyphenyl)-2-(2-hydroxyphenyl)propan (o,p-BPA), substituierte Indane, Hydroxyphenyl-indanole, Hydroxyphenyl-chromane, substituierte Xanthene und höher kondensierte Verbindungen mit drei oder mehr Phenylringen im Molekülgerüst.

Die genannten Nebenprodukte wie auch Wasser, Phenol und Aceton können die Eignung von BPA zur Herstellung von Polymeren beeinträchtigen und müssen durch geeignete Verfahren abgetrennt werden. Insbesondere zur Herstellung von Polycarbonat werden üblicherweise hohe Reinheitsanforderungen an den Rohstoff BPA gestellt.

Die Aufarbeitung und Reinigung von BPA erfolgt üblicherweise durch eine mehrstufige Kaskade von geeigneten Reinigungsverfahren wie beispielsweise Suspensionskristallisation, Schmelzekristallisation, Destillation und Desorption. In einer technisch bevorzugten Ausführungsform erfolgt die Abtrennung von BPA aus der Reaktionsmischung in Form eines etwa äquimolaren kristallinen Addukts mit Phenol durch Abkühlen der Reaktionsmischung unter Auskristallisieren des BPA/Phenol-Addukts. Die Kristallisation erfolgt bevorzugt als Suspensionskristallisation. Unter Suspensionskristallisation versteht man die Kristallisation aus einer Flüssigkeit, wobei die Kristalle mit der Flüssigkeit eine Suspension bilden. Die BPA/Phenol-Adduktkristalle werden anschließend durch eine geeignete Apparatur zur Fest-Flüssigtrennung wie Drehfilter oder Zentrifugen von der Flüssigphase abgetrennt und erforderlichenfalls der weiteren Reinigung zugeführt. So erhaltene Adduktkristalle weisen typischerweise eine Reinheit von größer als 99 Gew.-% BPA bezogen auf die Nebenkomponenten bei einem Phenolanteil von ca. 40 Gew.-% auf. Durch Waschen mit geeigneten Lösungen, die typischerweise eine oder mehrere Komponenten aus der Gruppe Aceton, Wasser, Phenol, BPA und Nebenkomponenten enthalten, können die Adduktkristalle von oberflächlich anhaftenden Verunreinigungen befreit werden.

Der bei der Fest-Flüssigtrennung anfallende Flüssigstrom (Mutterlauge) enthält Phenol, BPA, bei der Reaktion entstandenes Wasser, nicht umgesetztes Aceton und ist angereichert an den bei der BPA-Herstellung typischerweise anfallenden Nebenkomponenten. Dieser Mutterlaugenstrom wird in einer bevorzugten Ausführungsform in die Reaktionseinheit zurückgeführt. Um die katalytische Aktivität der sauren Ionentauscher aufrecht zu erhalten wird zuvor entstandenes Wasser bevorzugt durch Destillation entfernt, wobei auch gegebenenfalls noch vorhandenes Aceton aus der Mutterlauge entfernt wird. Der so erhaltene entwässerte Reaktionsstrom wird um Phenol und Aceton ergänzt und in die Reaktionseinheit zurückgeführt. Alternativ können auch vor Durchführung der Suspensionskristallisation des BPA-Phenol-Addukts Wasser und Aceton ganz oder teilweise destillativ entfernt werden. Bei den genannten Destillationsschritten kann auch eine Teilmenge des in der Reaktionslösung vorhandenen Phenols destillativ abgetrennt werden.

Bei einer derartigen Kreislauffahrweise tritt als Problem auf, dass Nebenprodukte der BPA-Herstellung im Kreislaufstrom angereichert werden und zur Desaktivierung des Katalysatorsystem führen können. Um eine übermäßige Anreicherung von Nebenkomponenten im Kreislaufstrom zu vermeiden, wird bevorzugt eine Teilmenge des Kreislaufstroms - gegebenenfalls nach teilweiser oder vollständiger destillativer Rückgewinnung von Phenol - aus der Prozesskette als BPA-Harz ausgeschleust.

Außerdem hat es sich als vorteilhaft erwiesen einen Teil oder die Gesamtmenge des Kreislaufstroms nach der Fest-Flüssigtrennung und vor oder nach der Abtrennung von Wasser und Restaceton über eine mit saurem lonentauscher befüllte Umlagerungseinheit zu führen. Diese Einheit wird im allgemeinen bei höheren Temperaturen betrieben als die Reaktionseinheit. In dieser Umlagerungseinheit werden unter den vorherrschenden Bedingungen einige der im Kreislaufstrom vorhandenen Nebenkomponenten der BPA-Herstellung zu BPA isomerisiert, so dass die Gesamtausbeute an BPA erhöht werden kann.

Die im Anschluss an die oben beschriebene Suspensionskristallisation der Reaktionslösung und Fest-Flüssig-Trennung erhaltenen BPA-Phenol-Adduktkristalle werden erforderlichenfalls weitergehenden Reinigungsschritten zugeführt, wobei die Abtrennung von Phenol und gegebenenfalls die Verringerung der Konzentration an Nebenkomponenten erzielt wird. Dabei wird bevorzugt der überwiegende Teil des Phenols abgetrennt.

So können die Adduktkristalle beispielsweise aus Phenol, aus organischen Lösungsmitteln, aus Wasser oder aus Mischungen der genannten Verbindungen gemäß einer Suspensionskristallisation umkristallisiert werden. Hierbei kann durch die Wahl geeigneter Lösungsmittel auch das in den Adduktkristallen vorhandene Phenol ganz oder teilweise abgetrennt werden. Das gegebenenfalls nach der Umkristallisation im BPA verbleibende Phenol kann anschließend durch geeignete destillative, desorptive oder extraktive Methoden gänzlich abgetrennt werden.

Alternativ kann auch zunächst Phenol aus den Adduktkristallen entfernt werden. Bevorzugte Methoden hierbei sind Desorption der Schmelze mit heißen Inertgasen, Vakuumdestillation oder eine Kombination der genannten Methoden. Auf diesem Wege ist es möglich aus den Adduktkristallen BPA mit einem Restphenolgehalt von weniger als 100 ppm zu gewinnen. Durch geeignete Reaktionsführung und gegebenenfalls Zugabe von Stabilisatoren kann erreicht werden, dass BPA unter der thermischen Belastung der destillativen oder desorptiven Phenolentfernung nicht in nennenswertem Umfang gespalten wird.

In Abhängigkeit von den Verfahrensbedingungen der Suspensionskristallisation aus der Reaktionslösung und der Durchführung der Fest-Flüssigtrennung und Kristallwäsche ist das nach Abtrennung von Phenol aus den Adduktkristallen erhaltene BPA zur Herstellung von polymeren Werkstoffen geeignet. Insbesondere zur Herstellung hochwertiger Werkstoffe wie Polycarbonat kann es nötig sein, das nach Abtrennung von Phenol erhaltene BPA einer weiteren Reinigungsoperation zuzuführen. Die Endreinigung kann erfolgen durch Suspensionskristallisation aus Wasser oder geeigneten organischen Lösungsmitteln, Schmelzekristallisation in Form einer statischen oder dynamischen Schichtkristallisation, Extraktion mit Wasser, wässrigen neutralen, sauren oder basischen Salzlösungen oder geeigneten organischen Lösungmitteln oder in Form einer ein- oder mehrstufigen Destillation. Durch die Durchführung der genannten Reinigungsoperationen oder einer geeigneten Kombination derselben ist es möglich, BPA mit einer Reinheit von größer als 99,9 Gew.-% zu erhalten, das zur Herstellung hochwertiger Polymerwerkstoff in besonderer Weise geeignet ist.

In einer bevorzugten Ausführungsform der vorliegenden Erfindung wird der mit Wasser fluidisierte Bisphenol-Staub in ein Gefäß überführt, in dem reines Phenol oder Phenol-Wasser-Gemische vorgelegt werden. Falls Phenol-Wasser-Gemische vorgelegt werden, bestehen diese bevorzugt aus einem Gemisch mit dem Massenverhältnis Phenol : Wasser = 9 : 1 bis 1 : 9.

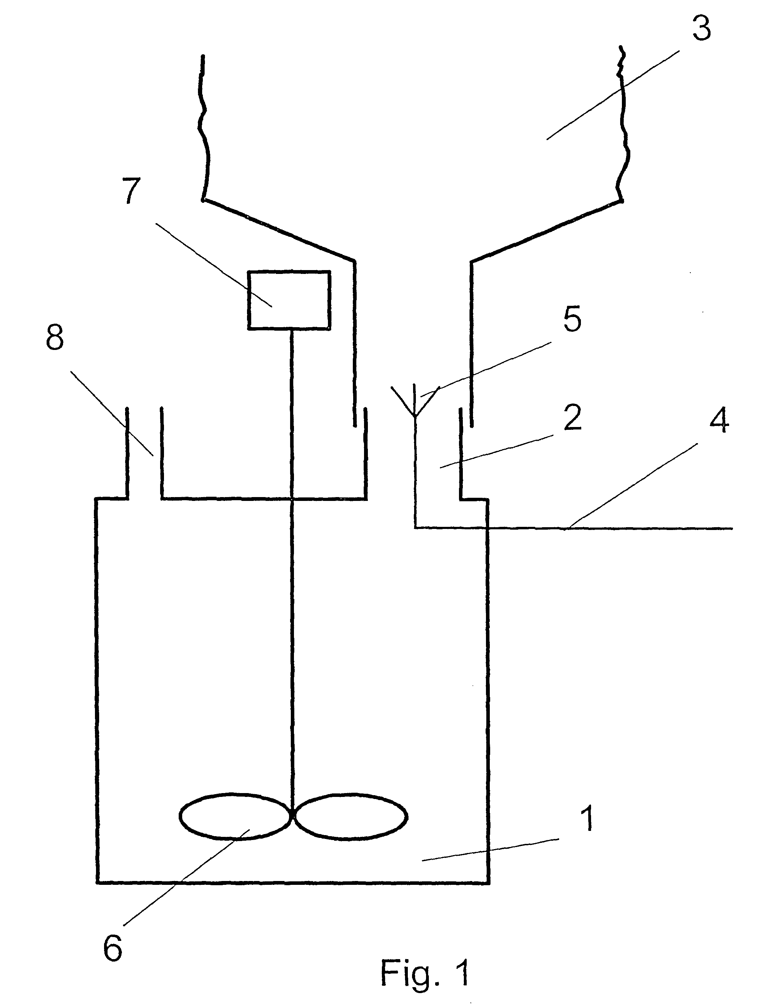

Die vorliegende Erfindung wird im folgenden anhand einer Zeichnung (Fig. 1) erläutert. Die Zeichnung stellt eine bevorzugte Ausführungsform der Erfindung dar. Die Erfindung ist nicht auf diese bevorzugte Ausführungsform beschränkt.

Der Bisphenol-A-Staub befindet sich in einem Vorratsgebinde 3. Dieses ist ein sackartiges Gebinde aus Kunststoffgewebe mit einem Füllvolumen von ca. 1 m³, das am Boden eine Auslassöffnung besitzt. Dieses Vorratsgebinde wird als "big bag" bezeichnet. Die Auslassöffnung am Boden des big bags ist über die Öffnung 2 des Vorratsbehälters 1 gestülpt. Über die Zuleitung 4, die in der Düse 5 endet, wird Wasser am unteren Ende des Vorratsgebindes 3 eingesprüht. Dadurch erfolgt eine Fluidisierung des Bisphenol-Staubes im Vorratsgebinde 3 der infolge dessen getrieben durch die Schwerkraft in den Vorratsbehälter 1 sinkt. Der Vorratsbehälter 1 ist mit einem Rührer 6 getrieben durch einen Motor 7 ausgerüstet durch den der fluidisierte Bisphenol-Staub homogenisiert werden kann. Über die Öffnung 8 wird dem System Stickstoff als Inertgas zugeführt.

### Beispiel 1 (erfindungsgemäß)

300 kg Bisphenol-A-Staub, der bei der Herstellung von Bisphenol-A-Prills entstand, befand sich in einem sackartigen Gebinde aus Kunststoffgewebe, das am Boden eine Öffnung besitzt (sogenannter big bag). Der big bag hatte ein Volumen von ca. 1 m³. In einer Vorrichtung gemäß Fig. 1 wurden insgesamt 801 Wasser über eine Düse in die Öffnung am Bodenende des big bags zugeführt. Bereits nach 5 Minuten war der big bag nahezu vollständig in die inertisierte Vorlage entleert. Nun wurden 1000 kg Phenol bei einer Temperatur von 65°C in die Vorlage gegeben und die Mantelbeheizung der Vorlage in Betrieb genommen. Das Rührwerk wurde eingeschaltet und der fluidisierte Bisphenol-A-Staub wurde vollständig gelöst. Die entstandene Lösung wurde direkt in den Verfahrensabschnitt Kristallisation der Herstellung von Bisphenol-A eingespeist.

### Beispiel 2 (Vergleichsbeispiel)

Versuch 1 wurde wiederholt ohne dass Wasser zugeführt wurde. Es wurde versucht durch mechanische Vorgänge (Rütteln, Schlagen an die big bag Wand, mechanisches Herausstoßen des Bisphenol-A-Staubes mittels Stangen durch den geöffneten big bag) den Bisphenol-A-Staub aus dem big bag durch die Bodenöffnung herauszufördern. Auch nach 30 Minuten intensiver Bearbeitung war ein Großteil des Bisphenol-A-Staubes im big bag verblieben.

## Patentansprüche

1. Verfahren zur Fluidisierung von Bisphenol-Staub umfassend die Zugabe von 0,1 bis 10 Gewichtsteilen Wasser pro 1 Gewichtsteil Bisphenol-Staub.

2. Verfahren nach Anspruch 1, wobei der Bisphenol-Staub Bisphenol-A-Staub ist.

3. Fluidisierter Bisphenol-Staub enthaltend 0,1 bis 10 Gewichtsteile Wasser je 1 Gewichtsteil Bisphenol-Staub.

4. Fluidisierter Bisphenol-A-Staub enthaltend 0,1 bis 10 Gewichtsteile Wasser je 1 Gewichtsteil Bisphenol-A-Staub.

5. Verfahren zur Herstellung von einem Bisphenol, wobei dem Verfahren vor, während oder nach der Reaktion eines Phenols mit einer Carbonylverbindung fluidisierter Bisphenol-Staub nach Anspruch 3 zugeführt wird.

6. Verfahren gemäß Anspruch 5, wobei der Bisphenol-Staub Bisphenol-A-Staub ist und das Phenol unsubstituiertes Phenol ist und die Carbonylverbindung Aceton ist.

## Claims

1. A process for fluidising bisphenol dust, consisting of adding 0.1 to 10 parts by weight of water per 1 part by weight of bisphenol dust.

2. A process according to Claim 1, wherein the bisphenol dust is bisphenol A dust.

3. Fluidised bisphenol dust containing 0.1 to 10 parts by weight of water per 1 part by weight of bisphenol dust.

4. Fluidised bisphenol A dust containing 0.1 to 10 parts by weight of water per 1 part by weight of bisphenol A dust.

5. A process for preparing a bisphenol, wherein fluidised bisphenol dust according to Claim 3 is supplied to the process before, during or after reaction of a phenol with a carbonyl compound.

6. A process according to Claim 5, wherein the bisphenol dust is bisphenol A dust and the phenol is unsubstituted phenol and the carbonyl compound is acetone.

## Revendications

1. Procédé de fluidisation de poudre de bisphénol comprenant l'addition de 0,1 à 10 parties en poids d'eau pour 1 partie en poids de poudre de bisphénol.

2. Procédé selon la revendication 1, dans lequel la poudre de bisphénol est de la poudre de bisphénol A.

3. Poudre de bisphénol fluidisée contenant 0,1 à 10 parties en poids d'eau pour chaque fois 1 partie en poids de poudre de bisphénol.

4. Poudre de bisphénol A fluidisée contenant 0,1 à 10 parties en poids d'eau pour 1 partie en poids de poudre de bisphénol A.

5. Procédé de préparation d'un bisphénol, dans lequel on amène au procédé avant, pendant ou après la réaction d'un phénol avec un composé carbonylé, la poudre de bisphénol fluidisée selon la revendication 3.

6. Procédé selon la revendication 5, dans lequel la poudre de bisphénol est de la poudre de bisphénol A et le phénol est un phénol non substitué et le composé carbonylé est de l'acétone.
